(19) Europäisches Patentamt / European Patent Office / Office européen des brevets

(11) **EP 3 559 287 B1**

(12) **EUROPEAN PATENT SPECIFICATION**

(45) Date of publication and mention of the grant of the patent:
**17.08.2022 Bulletin 2022/33**

(21) Application number: **17826579.9**

(22) Date of filing: **20.12.2017**

(51) International Patent Classification (IPC):
**C21D 11/00** $^{(2006.01)}$ **B21B 37/74** $^{(2006.01)}$
**B21B 37/76** $^{(2006.01)}$ **C21D 9/46** $^{(2006.01)}$
**C21D 9/48** $^{(2006.01)}$

(52) Cooperative Patent Classification (CPC):
**C21D 11/005; B21B 37/74; B21B 37/76; C21D 9/46; C21D 9/48; C21D 11/00**

(86) International application number:
**PCT/IB2017/058189**

(87) International publication number:
**WO 2018/116194 (28.06.2018 Gazette 2018/26)**

(54) **A METHOD OF DYNAMICAL ADJUSTMENT FOR MANUFACTURING A THERMALLY TREATED STEEL SHEET**

VERFAHREN ZUR DYNAMISCHEN ANPASSUNG ZUR HERSTELLUNG EINES THERMISCH BEHANDELTEN STAHLBLECHS

PROCÉDÉ DE RÉGLAGE DYNAMIQUE POUR LA FABRICATION D'UNE TÔLE D'ACIER TRAITÉE THERMIQUEMENT

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Validation States:
**MA**

(30) Priority: **20.12.2016 PCT/IB2016/001790**

(43) Date of publication of application:
**30.10.2019 Bulletin 2019/44**

(73) Proprietor: **ArcelorMittal**
**1160 Luxembourg (LU)**

(72) Inventor: **BONNET, Frédéric**
**54150 Avril (FR)**

(74) Representative: **Lavoix**
**2, place d'Estienne d'Orves**
**75441 Paris Cedex 09 (FR)**

(56) References cited:
EP-A1- 1 970 457     EP-A1- 2 287 345
WO-A1-2017/050311     US-A- 5 891 275
US-A1- 2003 089 431     US-A1- 2004 205 951
US-A1- 2005 016 712     US-A1- 2007 198 122
US-A1- 2009 265 146     US-A1- 2014 175 713
US-B1- 6 185 970

**EP 3 559 287 B1**

**Description**

The present invention relates to a method of dynamical adjustment for manufacturing a thermally treated steel sheet having a chemical steel composition and a microstructure $m_{target}$ comprising from 0 to 100% of at least one phase chosen among: ferrite, martensite, bainite, pearlite, cementite and austenite, in a heat treatment line.

It is known to use coated or bare steel sheets for the manufacture of automotive vehicles. A multitude of steel grades are used to manufacture a vehicle. The choice of steel grade depends on the final application of the steel part. For example, IF (Interstitial-Free) steels can be produced for an exposed part, TRIP (Transformation-Induced Plasticity) steels can be produced for seat and floor cross members or A-pillars and DP (Dual Phase) steels can be produced for rear rails or roof cross member.

During the production of theses steels, crucial treatments are performed on the steel in order to obtain the desired part having excepted mechanical properties for one specific application. Such treatments can be, for example, a continuous annealing before deposition of a metallic coating or a quenching and partitioning treatment. These treatments are performed in an adapted furnace line.

During these treatments, some unplanned deviations can appear online. For example, a temperature in the furnace, the thickness of the steel sheet, the line speed can vary.

The patent application US4440583 relates to a method of controlled cooling for steel strip implemented by use of a cooling apparatus comprising a plurality of nozzles disposed in the direction in which strip travels, the nozzles spraying coolant against the hot running strip, and a flow-rate control valve attached to the pipe that supplies the coolant to the nozzles. By using an equation containing the thickness of strip, the cooling starting and finishing temperatures, and the desired cooling rate, the heat transfer rate needed to obtain the desired cooling rate is calculated, and the obtained heat transfer rate is corrected according to the effect of natural cooling in idle-pass zones preceding and following the coolant spray zone. Then the flow rate of the coolant is derived, and set, from its pre-established relationship with the heat transfer rate. The length of the coolant spraying zone along the strip travel path is calculated using the running speed of the strip, the cooling starting and finishing temperatures, and the desired cooling rate. The nozzles are set to turn on and off so that coolant is sprayed from only such a number of nozzles as correspond to the calculated value. When strip thickness varies while controlled cooling is being effected, the heat transfer rate is re-calculated, on the basis of the above settings, to correct the coolant flow rate accordingly. When strip speed varies, the length of the coolant spraying region is re-calculated to correct the on-off pattern of the nozzles.

In this method, when a deviation appears, the heat transfer rate or the length of the coolant spraying region is re-calculated to correct the deviation. This method does not take into account the steel sheet characteristics comprising chemical composition, microstructure, properties, surface texture, etc. Thus, there is a risk that the same correction is applied to any kind of steel sheet even if each steel sheet has its own characteristics. The method allows for a non-personalized cooling treatment of a multitude of steel grades.

Consequently, the correction is not adapted to one specific steel and therefore at the end of the treatment, the desired properties are not obtained. Moreover, after the treatment, the steel can have a big dispersion of the mechanical properties. Finally, even if a wide range of steel grades can be manufactured, the quality of the treated steel is poor.

The patent application US 2009/265146 describes a method of modeling the time gradient of the state of a steel volume by a computer, the steel volume being modeled through a number of volume elements, which state at a specific time including variables for each of the volume elements w characteristic of an enthalpy present at this time in the respective volume element and that are characteristic for components in which the steel is present in the respective volume element at this time in the phases austenite, ferrite and cementite, the computer determining for at least one of the volume elements the time gradient of the characteristic variables by solving a thermal conductivity equation and a phase transition equation.

Thus, the object of the invention is to solve the above drawbacks by providing a method of dynamical adjustment for manufacturing a thermally treated steel sheet a having a specific chemical steel composition and a specific microstructure $m_{target}$ to reach in a heat treatment line. Another object is to adjust a thermal path online by providing a treatment adapted to each steel sheet, such treatment being calculated very precisely in the lowest calculation time possible. Another object is to provide a steel sheet having the excepted properties, such properties having the minimum of properties dispersion possible.

This object is achieved by providing a method according to claim 1. The method can also comprise any characteristics of claims 2 to 20.

Other characteristics and advantages of the invention will become apparent from the following detailed description of the invention.

To illustrate the invention, various embodiments and trials of non-limiting examples will be described, particularly with reference to the following Figures:

Figure 1 illustrates an example according to the present invention.

Figure 2 illustrates a continuous annealing of a steel sheet comprising a heating step, a soaking step, a cooling step and an overaging step.

Figure 3 illustrates a preferred embodiment according to the present invention.

Figure 4 illustrates one example according to the invention wherein a continuous annealing is performed on a steel sheet before the deposition of a coating by hot-dip.

The following terms will be defined:

- CC: chemical composition in percentage in weight percent,
- $m_{target}$: targeted value of the microstructure,
- $m_{standard}$: the microstructure of the selected product,
- Ptarget: targeted value of a mechanical property,
- $m_i$: initial microstructure of the steel sheet,
- X: phase fraction in weight percent,
- T: temperature in degree Celsius (°C),
- t: time (s),
- s: seconds,
- UTS: ultimate tensile strength (MPa),
- YS: yield stress (MPa),
- metallic coating based on zinc means a metallic coating comprising above 50% of zinc,
- metallic coating based on aluminum means a metallic coating comprising above 50% of aluminum and
- TT: thermal treatment and
- thermal path, TT, $TP_{target}$, $TP_x$ and $TP_{xint}$ comprises a time, a temperature of the thermal treatment and at least one rate chosen from: a cooling, an isotherm or a heating rate . The isotherm rate means a rate having a constant temperature and
- nanofluids: fluid comprising nanoparticles.

The designation "steel" or "steel sheet" means a steel sheet, a coil, a plate having a composition allowing the part to achieve a tensile strength up to 2500 MPa and more preferably up to 2000MPa. For example, the tensile strength is above or equal to 500 MPa, preferably above or equal to 1000 MPa, advantageously above or equal to 1500 MPa. A wide range of chemical composition is included since the method according to the invention can be applied to any kind of steel.

The invention relates to a method of dynamical adjustment for manufacturing a thermally treated steel sheet having a chemical steel composition and a microstructure $m_{target}$ comprising from 0 to 100% of at least one phase chosen among: ferrite, martensite, bainite, pearlite, cementite and austenite, in a heat treatment line wherein a predefined thermal treatment TT is performed on the steel sheet, such method comprising:

A. a control step wherein at least one sensor detects any deviation happening during the thermal treatment TT ,
B. a calculation step performed when a deviation is detected during the thermal treatment such that a new thermal path $TP_{target}$ is determined to reach $m_{target}$ taking the deviation into account, such calculation step comprising:

1) a calculation sub step wherein at least two thermal path, $TP_x$ corresponding to one microstructure $m_x$ at the end of $TP_x$, are calculated based on TT and the microstructure $m_i$ of the steel sheet to reach $m_{target}$,
2) a selection sub step wherein one new thermal path TPtarget to reach $m_{target}$ is selected, $TP_{target}$ being chosen from TPx and being selected such that $m_x$ is the closest to $m_{target}$,

C. a new thermal treatment step wherein TPtarget is performed online on the steel sheet.

Without willing to be bound by any theory, it seems that when the method according to the present invention is applied, it is possible to correct any deviation happening during a thermal treatment by providing a personalized heat treatment depending on each steel sheet. To do so, a precise and specific new thermal path $TP_{target}$ is calculated in a short calculation time taking into account $m_{target}$, in particular the proportion of all the phases along the treatment, $m_i$ (including the microstructure dispersion along the steel sheet) and the deviation. Indeed, the method according to the present invention takes into account for the calculation the thermodynamically stable phases, i.e. ferrite, austenite, cementite and pearlite, and the thermodynamic metastable phases, i.e. bainite and martensite. Thus, a steel sheet having the expected properties with the minimum of properties dispersion possible is obtained.

Preferably, the microstructures $m_x$, $m_{target}$ and $m_i$ phases are defined by at least one element chosen from: the size, the shape and the chemical composition.

Preferably, the microstructure $m_{target}$ to reach comprises:

- 100% of austenite,
- from 5 to 95% of martensite, from 4 to 65% of bainite, the balance being ferrite,
- from 8 to 30% of residual austenite, from 0.6 to 1.5% of carbon in solid solution, the balance being ferrite, martensite, bainite, pearlite and/or cementite,
- from 1% to 30% of ferrite and from 1% to 30% of bainite, from 5 and 25% of austenite, the balance being martensite ,
- from 5 to 20% of residual austenite, the balance being martensite,
- ferrite and residual austenite,
- residual austenite and intermetallic phases,
- from 80 to 100% of martensite and from 0 to 20% of residual austenite
- 100% martensite,
- from 5 to 100% of pearlite and from 0 to 95% of ferrite and
- at least 75% of equiaxed ferrite, from 5 to 20% of martensite and bainite in amount less than or equal to 10%.

Advantageously, the steel sheets can be any kind of steel grade including Dual Phase DP, Transformation Induced Plasticity (TRIP), Quenched & Partitioned steel (Q&P), Twins Induced Plasticity (TWIP), Carbide Free Bainite (CFB), Press Hardening Steel (PHS), TRIPLEX, DUPLEX and Dual Phase High Ductility (DP HD).

The chemical composition depends on each steel sheet. For example, the chemical composition of a DP steel can comprise:

$0.05 < C < 0.3\%$,
$0.5 \leq Mn < 3.0\%$,
$S \leq 0.008\%$,
$P \leq 0.080\%$,
$N \leq 0.1\%$,
$Si \leq 1.0\%$,

the remainder of the composition making up of iron and inevitable impurities resulting from the development.

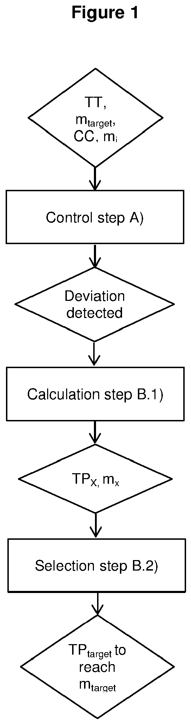

Figure 1 illustrates an example according to the invention wherein a TT is performed on a steel sheet in a heat treatment line, such steel sheet having a chemical composition CC and $m_{target}$ to reach.

According to the present invention in step A), any deviation happening during the thermal treatment is detected. Preferably, the deviation is due to a variation of a process parameter chosen from among: a furnace temperature, a steel sheet temperature, an amount of gas, a gas composition, a gas temperature, a line speed, a failure in the heat treatment line, a variation of the hot-dip bath, a steel sheet emissivity and a variation of the steel thickness.

A furnace temperature can be a heating temperature, a soaking temperature, a cooling temperature, an overaging temperature, in particular in a continuous annealing.

A steel sheet temperature can be measured at any time of the heat treatment in different positions of the heat treatment line, for example:

- in a heating section preferably being a direct flame furnace (DFF), a radian tube furnace (RTF), an electrical resistance furnace or an induction furnace,
- in cooling section, in particular, in jets cooling, in a quenching system or in a snout and
- in isothermal section preferably being an electrical resistance furnace.

To detect a temperature variation, the sensor can be a pyrometer or a scanner.

Usually, heat treatments can be performed in an oxidizing atmosphere, i.e. an atmosphere comprising an oxidizing gas being for example: $O_2$, $CH_4$, $CO_2$ or CO. They also can be performed in a neutral atmosphere, i.e. an atmosphere comprising a neutral gas being for example: $N_2$, Ar or He. Finally, they also can be performed in a reducing atmosphere, i.e. an atmosphere comprising a reducing gas being for example: $H_2$ or $HN_x$.

The variation of gas amount can be detected by barometer.

The line speed can be detected by a laser sensor.

For example, a failure in the heat treatment line can be:

- in a direct flame furnace: a burner not working anymore,
- in a radiant tube furnace: a radiant tube not working anymore,
- in an electrical furnace: a resistance not working anymore or
- in a cooling section: one or several jets cooling not working anymore.

In such cases, sensor can be a pyrometer, a barometer, an electrical consumption or a camera.

The variation of the steel thickness can be detected by a laser or an ultrasound sensor.

When a deviation is detected, at least two thermal path $TP_x$, corresponding to $m_x$, are calculated based on TT and $m_i$ to reach $m_{target}$, such $TP_x$ taking into account the deviation. The calculation of $TP_x$ is based on the thermal behavior and metallurgical behavior of the steel sheet compared to the conventional methods wherein only the thermal behavior is considered.

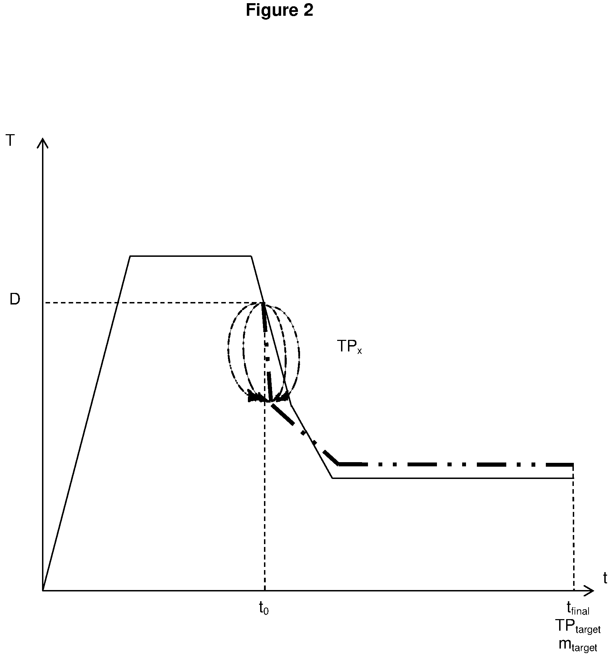

Figure 2 illustrates a continuous annealing of a steel sheet comprising a heating step, a soaking step, a cooling step and an overaging step. A deviation D due to a variation of $T_{soaking}$ is detected. Thus, a multitude of $TP_x$ is calculated to reach $m_{target}$ as shown only for the first cooling step in Figure 2. In this example, the calculated $TP_x$ also includes the second cooling step and the overaging step (not shown).

Preferably, at least 10 $TP_x$ are calculated, more preferably at least 50, advantageously at least 100 and more preferably at least 1000. For example, the number of calculated $TP_x$ is between 2 and 10000, preferably between 100 and 10000 and preferably between 1000 and 10000.

In step B.2), one new thermal path $TP_{target}$ to reach $m_{target}$ is selected. $TP_{target}$ is chosen from $TP_x$ and being selected such that $m_x$ is the closest to $m_{target}$. Thus, in Figure 1, $TP_{target}$ is chosen from a multitude of $TP_x$. Preferably, the differences between phases proportions of each phase present in $m_{target}$ and $m_x$ is $\pm$ 3%.

Advantageously, in step B.1), the thermal enthalpy H released or consumed between $m_i$ and $m_{target}$ is calculated such that:

$$H_x = (X_{ferrite} \ast H_{ferrite}) + (X_{martensite} \ast H_{martensite}) + (X_{bainite} \ast H_{bainite}) + (X_{pearlite} \ast H_{pearlite}) + (H_{cementite} + X_{cementite}) + (H_{austenite} + X_{austenite})$$

X being a phase fraction.

Without willing to be bound by any theory, H represents the energy released or consumed along the all thermal path when a phase transformation is performed. It is believed that some phase transformations are exothermic and some of them are endothermic. For example, the transformation of ferrite into austenite during a heating path is endothermic whereas the transformation of austenite into pearlite during a cooling path is exothermic. Preferably, $H_x$ is taken in account in the calculation of $TP_x$.

In a preferred embodiment, in step B.1), the all thermal cycle $TP_x$ is calculated such that:

$$T(t + \Delta t) = T(t) + \frac{(\varphi_{Convection} + \varphi_{radiance})}{\rho \cdot Ep \cdot C_{pe}} \Delta t \pm \frac{Hx}{C_{pe}}$$

with Cpe: the specific heat of the phase ($J \cdot kg^{-1} \cdot K^{-1}$), p: the density of the steel ($g.m^{-3}$), Ep: the thickness of the steel (m), $\varphi$: the heat flux (convective and radiative in W), $H_x$($J.kg^{-1}$), T: temperature (°C) and t: time (s).

Preferably in step B.1), at least one intermediate steel microstructure $m_{xint}$ corresponding to an intermediate thermal path $TP_{xint}$ and the thermal enthalpy $H_{xint}$ are calculated. In this case, the calculation of $TP_x$ is obtained by the calculation of a multitude of $TP_{xint}$. Thus preferably, $TP_x$ is the sum of all $TP_{xint}$ and $H_x$ is the sum of all $H_{xint}$. In this preferred embodiment, $TP_{xint}$ is calculated periodically. For example, it is calculated every 0.5 seconds, preferably 0.1 seconds or less.

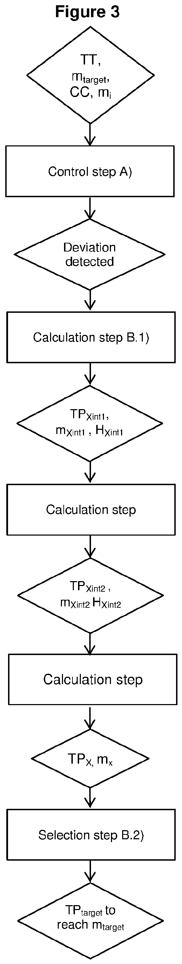

Figure 3 illustrates a preferred embodiment wherein in step B.1), $m_{int1}$ and $m_{int2}$ corresponding respectively to $TP_{xint1}$ and $TP_{xint2}$ as well as $H_{xint1}$ and $H_{xint2}$ are calculated. $H_x$ during the all thermal path is determined to calculate $TP_x$. according to the present invention, a multitude, i.e more than 2, of $TP_{xint}$, $m_{xint}$ and $H_{xint}$ are calculated to obtain $TP_x$ (not shown).

In a preferred embodiment, before step B.2), at least one targeted mechanical property Ptarget chosen among yield strength YS, Ultimate Tensile Strength UTS, elongation hole expansion, formability is selected. In this embodiment, preferably, $m_{target}$ is calculated based on $P_{target}$.

Without willing to be bound by any theory, it is believed that the characteristics of the steel sheet are defined by the process parameters applied during the steel production. Thus, advantageously, in step B.1), the process parameters undergone by the steel sheet before entering the heat treatment line are taken into account to calculate $TP_x$. For example, the process parameters comprise at least one element chosen from among: a cold rolling reduction rate, a coiling temperature, a run out table cooling path, a cooling temperature and a coil cooling rate.

In another embodiment, the process parameters of the treatment line that the steel sheet will undergo in the heat treatment line are taken into account to calculate $TP_x$. For example, the process parameters comprise at least one element chosen from among: a specific thermal steel sheet temperature to reach, the line speed, cooling power of the cooling sections, heating power of the heating sections, an overaging temperature, a cooling temperature, a heating temperature and a soaking temperature.

Preferably, the thermal path, $TP_x$, $TP_{xint}$, TT or TPtarget comprise at least one treatment chosen from: a heating, an isotherm or a cooling treatment. For example, the thermal path can be a recrystallization annealing, a press hardening path, a recovery path, an intercritical or full austenitic annealing, a tempering path, a partitioning path, isothermal path or a quenching path.

Preferably, a recrystallization annealing is performed. The recrystallization annealing comprises optionally a pre-heating step, a heating step, a soaking step, a cooling step and optionally an equalizing step. In this case, it is performed in a continuous annealing furnace comprising optionally a pre-heating section, a heating section, a soaking section, a cooling section and optionally an equalizing section. Without willing to be bound by any theory, it is believed that the recrystallization annealing is the thermal path the more difficult to handle since it comprises many steps to take into account comprising cooling and heating steps.

Advantageously, every time a new steel sheet enters into the heat treatment line, a new calculation step B.1) is automatically performed. Indeed, the method according to the present invention adapts the thermal path TPtarget to each steel sheet even if the same steel grade enters in the heat treatment line since the real characteristics of each steel often differs. The new steel sheet can be detected and the new characteristics of the steel sheet are measured and are preselected beforehand. For example, a sensor detects the welding between two coils.

Preferably, the adaptation of the thermal path is performed as the steel sheet entries into the heat treatment line on the first meters of the sheet in order to avoid strong process variation.

Preferably, an automatic calculation is performed during the thermal treatment to check if any deviation had appeared. In this embodiment, periodically, a calculation is realized to verify if a slight deviation had occurred. Indeed, the detection threshold of sensor is sometimes too high which means that a slight deviation is not always detected. The automatic calculation, performed for example every few seconds, is not based on a detection threshold. Thus, if the calculation leads to the same thermal treatment, i.e. the thermal treatment performs online, TT will not change. If the calculation leads to a different treatment due to a slight deviation, the treatment will change.

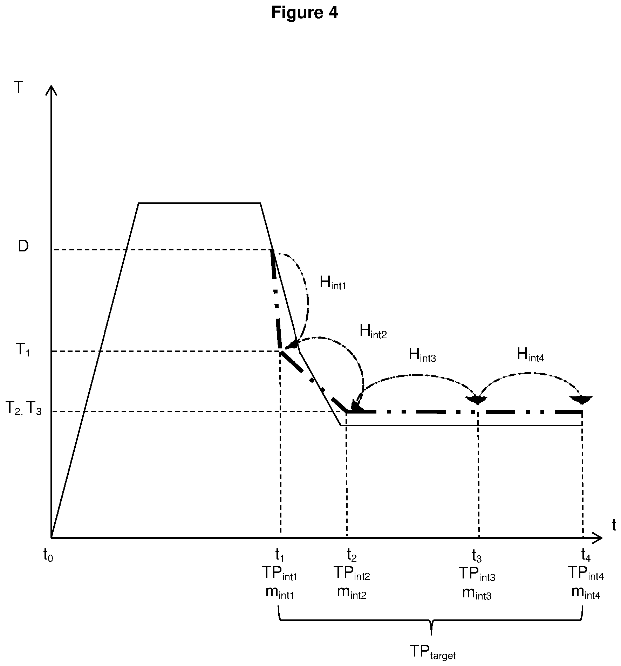

Figure 4 illustrates one example according to the invention wherein a continuous annealing is performed on a steel sheet before the deposition of a coating by hot-dip. With the method according to the present invention, when a deviation D appears, $TP_x$ is calculated based on $m_i$, the selected product, TT and $m_{target}$. In this example, intermediate thermal paths $TP_{xint1}$ to $TP_{xint4}$, corresponding respectively $m_{xint1}$ to $m_{xint4}$, and $H_{xint1}$ to $H_{xint4}$ are calculated. $H_x$ is determined in order to obtain $TP_x$. In this Figure, the represented TPtarget has been chosen from $TP_x$.

With the method according to the present invention, when a deviation appears, a new thermal treatment step comprising TPtarget is performed on the steel sheet in order to reach $m_{target}$.

Thus, a coil made of a steel sheet including said predefined product types include DP, TRIP, Q&P, TWIP, CFB, PHS, TRIPLEX, DUPLEX, DP HD is obtained, such coil having a standard variation of mechanical properties below or equal to 25MPa, preferably below or equal to 15MPa, more preferably below or equal to 9 MPa, between any two points along the coil. Indeed, without willing to be bound by any theory, it is believed that the method including the calculation step B.1) takes into account the microstructure dispersion of the steel sheet along the coil. Thus, TPtarget applied on the steel sheet allows for a homogenization of the microstructure and also of the mechanical properties.

The low value of standard variation is due to the precision of $TP_{target}$. Preferably, the mechanical properties are chosen from YS, UTS or elongation.

Preferably, the coil is covered by a metallic coating based on zinc or based on aluminum.

Preferably, in an industrial production, the standard variation of mechanical properties between 2 coils made of a steel sheet including said predefined product types include DP, TRIP, Q&P, TWIP, CFB, PHS, TRIPLEX, DUPLEX, DP HD measured successively produced on the same line is below or equal to 25MPa, preferably below or equal to 15MPa, more preferably below or equal to 9 MPa.

A thermally treatment line for the implementation of a method according to the present invention is used to perform TPtarget. For example, the thermally treatment line is a continuous annealing furnace, a press hardening furnace, a batch annealing or a quenching line.

Finally, the present invention can be implemented using a Computer program product comprising at least a metallurgical module, a thermal module and an optimization module that cooperate together to determine TPtarget such modules comprising software instructions that when implemented by a computer implement the method according to the present invention.

The metallurgical module predicts the microstructure ($m_x$, $m_{target}$ including metastable phases: bainite and martensite and stables phases: ferrite, austenite, cementite and pearlite) and more precisely the proportion of phases all along the treatment and predicts the kinetic of phases transformation.

The thermal module predicts the steel sheet temperature depending on the installation used for the thermal treatment, the installation being for example a continuous annealing furnace, the geometric characteristics of the band, the process parameters including the power of cooling, heating or isotherm power, the dynamic thermal enthalpy H released or consumed along the all thermal path when a phase transformation is performed.

The optimization module determines the best thermal path to reach $m_{target}$, i.e. TPtarget following the method according to the present invention using the metallurgical and thermal modules.

Examples

In the following examples, DP780GI having the following chemical composition was chosen:

| C (%) | Mn (%) | Si (%) | Cr (%) | Mo (%) | P (%) | Cu (%) | Ti (%) | N (%) |
|-------|--------|--------|--------|--------|-------|--------|--------|-------|
| 0.145 | 1.8 | 0.2 | 0.2 | 0.0025 | 0.015 | 0.02 | 0.025 | 0.06 |

The cold-rolling had a reduction rate of 55% to obtain a thickness of 1.2mm.

$m_{target}$ to reach comprised 12% of martensite, 58% of ferrite and 30% of bainite, corresponding to the following Ptarget : YS of 460MPa and UTS of 790MPa. A cooling temperature $T_{cooling}$ of 460°C has also to be reached in order to perform a hot-dip coating with a zinc bath. This temperature must be reached with an accuracy of +/- 2°C to guarantee good coatability in the Zn bath.

The thermal treatment TT to perform on the steel sheet is as follows:

- a pre-heating step wherein the steel sheet is heated from ambient temperature to 680°C during 37.5 seconds,
- a heating step wherein the steel sheet is heated from 680°C to 780°C during 40 seconds,
- soaking step wherein the steel sheet is heated at a soaking temperature $T_{soaking}$ of 780°C during 24.4seconds,
- a cooling step wherein the steel sheet is cooled with 11 jets cooling spraying $HN_x$ as follows:

| Jets | Jet 1 | Jet 2 | Jet 3 | Jet 4 | Jet 5 | Jet 6 | Jet 7 | Jet 8 | Jet 9 | Jet 10 | Jet 11 |
|------|-------|-------|-------|-------|-------|-------|-------|-------|-------|--------|--------|
| Cooling rate (°C/s) | 10 | 10 | 9 | 5 | 9 | 22 | 50 | 18 | 18 | 21 | 11 |
| Time (s) | 1.89 | 1.89 | 1.89 | 1.89 | 1.68 | 1.8 | 1.8 | 1.63 | 1.63 | 1.63 | 1.63 |
| T(°C) | 754 | 734 | 718 | 708 | 693 | 653 | 563 | 533 | 504 | 481 | 463 |
| Cooling power(%) | 0 | 0 | 0 | 0 | 0 | 0 | 28 | 100 | 100 | 100 | 100 |

- a hot-dip coating in a zinc bath à 460°C,
- the cooling of the steel sheet until the top roll during 27.8s at 300°C and
- the cooling of the steel sheet at ambient temperature.

Example 1: deviation of $T_{soaking}$

When the soaking temperature $T_{soaking}$ decreased from 780°C to 765°C, a new thermal path $TP_{target1}$ is determined to reach $m_{target}$ taking the deviation into account. To this end, a multitude of thermal path $TP_x$ was calculated based on TT, $m_i$ of DP780GI to reach $m_{target}$ and the deviation.

After the calculation of $TP_x$, one new thermal path $TP_{target1}$ to reach $m_{target}$ was selected, TPtargeti being chosen from $TP_x$ and being selected such that mx is the closest to $m_{target}$. $TP_{target1}$ cis as follows:

- a soaking step wherein the steel sheet is heated at a soaking temperature $T_{soaking}$ of 765°C during 24.4 seconds due to a deviation in the soaking section of the heat treatment line,
- a cooling step wherein the steel sheet is cooled with 11 jets cooling spraying $HN_x$ as follows:

| Jets | Jet 1 | Jet 2 | Jet 3 | Jet 4 | Jet 5 | Jet 6 | Jet 7 | Jet 8 | Jet 9 | Jet 10 | Jet 11 |
|------|-------|-------|-------|-------|-------|-------|-------|-------|-------|--------|--------|
| Cooling rate (°C/s) | 9 | 9 | 10 | 15 | 32 | 28 | 31 | 11 | 10 | 7 | 8 |
| Time (s) | 1.89 | 1.89 | 1.89 | 1.89 | 1.68 | 1.8 | 1.8 | 1.63 | 1.63 | 1.63 | 1.63 |
| T(°C) | 742 | 725 | 706 | 679 | 625 | 574 | 518 | 500 | 483 | 472 | 459 |
| Cooling power(%) | 0 | 0 | 0 | 25 | 50 | 50 | 45 | 45 | 45 | 45 | 45 |

- a hot-dip coating in a zinc bath à 460°C,

- the cooling of the steel sheet until the top roll during 27.8s at 300°C and
- the cooling of the steel sheet at ambient temperature.

Example 2: steel sheet having a different composition

A new steel sheet DP780GI entered into the heat treatment line so a calculation step was automatically performed based on the following new CC:

| C(%) | Mn(%) | Si(%) | Cr(%) | Mo(%) | P(%) | Cu(%) | Ti(%) | N(%) |
|------|-------|-------|-------|-------|------|-------|-------|------|
| 0.153 | 1.830 | 0.225 | 0.190 | 0.0025 | 0.015 | 0.020 | 0.025 | 0.006 |

The new thermal path $TP_{target2}$ was determined to reach $m_{target}$ taking the new CC into account. $TP_{target2}$ is as follows:
- a pre-heating step wherein the steel sheet is heated from ambient temperature to 680°C during 37.5 seconds,
- a heating step wherein the steel sheet is heated from 680°C to 780°C during 40 seconds,
- a soaking step wherein the steel sheet is heated at a soaking temperature $T_{soaking}$ of 780°C during 24.4 seconds,
- a cooling step wherein the steel sheet is cooled with 11 jets cooling spraying $HN_x$

| Jets | Jet 1 | Jet 2 | Jet 3 | Jet 4 | Jet 5 | Jet 6 | Jet 7 | Jet 8 | Jet 9 | Jet 10 | Jet 11 |
|------|-------|-------|-------|-------|-------|-------|-------|-------|-------|--------|--------|
| Cooling rate (°C/s) | 17 | 17 | 9 | 6 | 6 | 6 | 38 | 30 | 18 | 17 | 10 |
| Time (s) | 2.2 | 2.2 | 2.2 | 2.2 | 1.96 | 2.1 | 2.1 | 1.9 | 1.9 | 1.9 | 1.9 |
| T(°C) | 737 | 705 | 688 | 677 | 667 | 655 | 586 | 537 | 508 | 481 | 464 |
| Cooling power(%) | 100 | 100 | 30 | 0 | 0 | 0 | 100 | 100 | 100 | 100 | 100 |

- a hot-dip coating in a zinc bath à 460°C,
- the cooling of the steel sheet until the top roll during 26.8s at 300°C and
- the cooling of the steel sheet at ambient temperature.

Table 1 shows the steel properties obtained with TT, $TP_{target1}$ and $TP_{target2}$:

| | TT | $TP_{target1}$ | $TP_{target2}$ | Expected properties |
|---|---|---|---|---|
| $T_{cooling}$ obtained (°C) | 461 | 458 | 462 | 460 |
| Microstructure obtained at the end of the thermal path | $X_{martensite}$: 12% $X_{ferrite}$: 55% $X_{bainite}$: 33% | $X_{martensite}$: 12% $X_{ferrite}$: 61% $X_{bainite}$: 27% | $X_{martensite}$: 14% $X_{ferrite}$:55% $X_{bainite}$: 32% | $X_{martensite}$: 12% $X_{ferrite}$:58% $X_{bainite}$: 30% |
| Deviation (écart) with respect to $m_{target}$ | $X_{martensite}$: 0% $X_{ferrite}$: 3% $X_{bainite}$: 3% | $X_{martensite}$: 0% $X_{ferrite}$: 3% $X_{bainite}$: 3% | $X_{martensite}$: 2% $X_{ferrite}$:3% $X_{bainite}$: 2% | |
| YS (MPa) | 453.5 | 465 | 462 | 460 |
| YS deviation with respect to $P_{target}$ (MPa) | 6.5 | 5 | 2 | - |
| UTS (MPa) | 786.8 | 790 | 804 | 790 |
| UTS deviation with respect to $P_{target}$ (MPa) | 3.2 | 0 | 14 | - |

With the method according to the present invention, it is possible to adjust a thermal TT when a deviation appears or when a new steel sheet having a different CC enters into the heat treatment line. By applying the new thermal paths $TP_{target1}$ and $TP_{target2}$, it is possible to obtain a steel sheet having the desired expected properties, each TPtarget being precisely adapted to each deviation.

**Claims**

1. A method of dynamical adjustment for manufacturing a thermally treated steel sheet having a chemical steel composition and a microstructure $m_{target}$ comprising from 0 to 100% of at least one phase chosen among: ferrite, martensite, bainite, pearlite, cementite and austenite, in a heat treatment line wherein a predefined thermal treatment TT is performed on the steel sheet, such method comprising:

   A. a control step wherein at least one sensor detects any deviation happening during the thermal treatment,
   B. a calculation step performed when a deviation is detected during the thermal treatment such that a new thermal path $TP_{target}$ is determined to reach $m_{target}$ taking the deviation into account, such calculation step comprising:

      1) a calculation sub step wherein at least two thermal path, $TP_x$ corresponding to one microstructure $m_x$ obtained at the end of $TP_x$, are calculated based on TT and the microstructure $m_i$ of the steel sheet to reach $m_{target}$,
      2) a selection sub step wherein one new thermal path TPtarget to reach $m_{target}$ is selected, $TP_{target}$ being chosen from TPx and being selected such that $m_x$ is the closest to $m_{target}$,

   C. a new thermal treatment step wherein TPtarget is performed online on the steel sheet.

2. Method according to claim 1, wherein in step A), the deviation is due to a variation of one process parameter chosen from among: a furnace temperature, a steel sheet temperature, an amount of gas, a gas composition, a gas temperature, a line speed, a failure in the heat treatment line, a variation of the hot-dip bath, a steel sheet emissivity and a variation of the steel thickness.

3. Method according to anyone of claims 1 to 2, wherein the phases are defined by at least one element chosen from: the size, the shape and the chemical composition.

4. Method according to anyone of claims 1 to 3, wherein the microstructure $m_{target}$ comprises:

   - 100% of austenite,
   - from 5 to 95% of martensite, from 4 to 65% of bainite, the balance being ferrite,
   - from 8 to 30% of residual austenite, from 0.6 to 1.5% of carbon in solid solution, the balance being ferrite, martensite, bainite, pearlite and/or cementite,
   - from 1% to 30% of ferrite and from 1% to 30% of bainite, from 5 and 25% of austenite, the balance being martensite ,
   - from 5 to 20% of residual austenite, the balance being martensite,
   - ferrite and residual austenite,
   - residual austenite and intermetallic phases,
   - from 80 to 100% of martensite and from 0 to 20% of residual austenite
   - 100% martensite,
   - from 5 to 100% of pearlite and from 0 to 95% of ferrite and
   - at least 75% of equiaxed ferrite, from 5 to 20% of martensite and bainite in amount less than or equal to 10%.

5. Method according to anyone of claims 1 to 4, wherein the steel sheet can be Dual Phase, Transformation Induced Plasticity, Quenched & Partitioned steel, Twins Induced Plasticity, Carbide Free Bainite, Press Hardening Steel, TRIPLEX, DUPLEX and Dual Phase High Ductility.

6. Method according to anyone of claims 1 to 5, the differences between phases proportions of phase present in $m_{target}$ and $m_x$ is $\pm$ 3%.

7. Method according to anyone of claims 1 to 6, wherein in step B.1), the thermal enthalpy H released or consumed between $m_i$ and $m_{target}$ is calculated such that:

$$H_x = (X_{ferrite} * H_{ferrite}) + (X_{martensite} * H_{martensite}) + (X_{bainite} * H_{bainite}) + (X_{pearlite} * H_{pearlite}) + (H_{cementite} + X_{cementite}) + (H_{austenite} + X_{austenite})$$

X being a phase fraction.

8. Method according to claim 7, wherein in step B.1), the all thermal cycle $TP_x$ is calculated such that:

$$T(t + \Delta t) = T(t) + \frac{(\varphi_{Convection} + \varphi_{radiance})}{\rho \cdot Ep \cdot C_{pe}} \Delta t \pm \frac{Hx}{C_{pe}}$$

with Cpe: the specific heat of the phase ($J \cdot kg^{-1} \cdot K^{-1}$), p: the density of the steel ($g.m^{-3}$), Ep: thickness of the steel (m), $\varphi$: the heat flux (convective + radiative in W), $H_x$ ($J.kg^{-1}$), T: temperature (°C) and t: time (s).

9. Method according to anyone of claims 7 or 8, wherein in step B.1), at least one intermediate steel microstructure $m_{xint}$ corresponding to an intermediate thermal path $TP_{xint}$ and the thermal enthalpy $H_{xint}$ are calculated.

10. Method according to claim 9, wherein in step in step B.1), $TP_x$ is the sum of all $TP_{xint}$ and $H_x$ is the sum of all $H_{xint}$.

11. Method according to anyone of claims 1 to 10, wherein before step B.1), at least one targeted mechanical property Ptarget chosen among yield strength YS, Ultimate Tensile Strength UTS, elongation hole expansion, formability is selected.

12. Method according to claim 11, wherein $m_{target}$ is calculated based on $P_{target}$.

13. Method according to anyone of the preceding claims wherein in step B.1), the process parameters undergone by the steel sheet before entering the heat treatment line are taken into account to calculate $TP_x$.

14. Method according to claim 13, wherein the process parameters comprise at least one element chosen from among: a cold rolling reduction rate, a coiling temperature, a run out table cooling path, a cooling temperature and a coil cooling rate.

15. Method according to anyone of claims 1 to 14, wherein in step B.1), the process parameters of the treatment line that the steel sheet will undergo in the heat treatment line are taken into account to calculate $TP_x$.

16. Method according to claim 15, wherein the process parameters comprise at least one element chosen from among: a specific thermal steel sheet temperature to reach, the line speed, cooling power of the cooling sections, heating power of the heating sections, an overaging temperature, a cooling temperature, a heating temperature and a soaking temperature.

17. A method according to anyone of the preceding claims, wherein the thermal path, $TP_x$, $TP_{xint}$, TT or TPtarget comprise at least one treatment chosen from: a heating, an isotherm or a cooling treatment.

18. A method according to anyone of claims 1 to 17, wherein every time a new steel sheet enters into the heat treatment line, a new calculation step B.1) is automatically performed.

19. A method according to claim 18, wherein an adaptation of the thermal path is performed as the steel sheet entries into the heat treatment line on the first meters of the sheet.

20. A method according to anyone of claims 1 to 19, wherein an automatic calculation is performed during the thermal treatment to check if any deviation had appeared.

**Patentansprüche**

1. Verfahren zur dynamischen Einstellung zum Herstellen eines wärmebehandelten Stahlblechs, das eine chemische Stahlzusammensetzung und eine Mikrostruktur $m_{Ziel}$ aufweist, umfassend 0 bis 100 % mindestens einer Phase, die ausgewählt ist aus: Ferrit, Martensit, Bainit, Perlit, Zementit und Austenit, in einer Wärmebehandlungslinie, in der eine vordefinierte Wärmebehandlung TT an dem Stahlblech ausgeführt wird, das Verfahren umfassend:

A. einen Kontrollschritt, bei dem mindestens ein Sensor jede Abweichung erfasst, die während der thermischen Behandlung auftritt,

B. einen Berechnungsschritt, der ausgeführt wird, wenn eine Abweichung während der thermischen Behandlung festgestellt wird, sodass ein neuer thermischer Pfad $TP_{Ziel}$ bestimmt wird, um $m_{Ziel}$ unter Berücksichtigung der Abweichung zu erreichen, dieser Berechnungsschritt umfassend:

1) einen Berechnungsunterschritt, wobei mindestens zwei thermische Pfade $TP_x$, die einer Mikrostruktur $m_x$ entsprechen, die am Ende von $TP_x$ erlangt wird, basierend auf TT und der Mikrostruktur $m_i$ des Stahlblechs berechnet werden, um $m_{Ziel}$ zu erreichen,

2) einen Auswahlunterschritt, wobei ein neuer thermischer Pfad $TP_{Ziel}$ zum Erreichen von $m_{Ziel}$ ausgewählt wird, wobei $TP_{Ziel}$ ausgewählt ist aus $TP_x$ und ausgewählt ist, sodass $m_x$ am nächsten zu $m_{Ziel}$ ist,

C. einen neuen Wärmebehandlungsschritt, wobei $TP_{Ziel}$ online auf dem Stahlblech ausgeführt wird.

2. Verfahren nach Anspruch 1, wobei in Schritt A) die Abweichung auf eine Veränderung eines Prozessparameters zurückzuführen ist, der ausgewählt ist aus: einer Ofentemperatur, einer Stahlblechtemperatur, einer Gasmenge, einer Gaszusammensetzung, einer Gastemperatur, einer Liniengeschwindigkeit, einer Störung in der Wärmebehandlungslinie, einer Veränderung des Schmelztauchbads, einem Stahlblech-Emissionsgrad und einer Veränderung der Stahlstärke.

3. Verfahren nach einem der Ansprüche 1 bis 2, wobei die Phasen durch mindestens ein Element definiert sind, ausgewählt aus: der Größe, der Form und der chemischen Zusammensetzung.

4. Verfahren nach einem der Ansprüche 1 bis 3, wobei die Mikrostruktur $m_{Ziel}$ Folgendes umfasst:

- 100 % Austenit,
- 5 bis 95 % Martensit, 4 bis 65 % Bainit, wobei der Rest Ferrit ist,
- 8 bis 30 % Restaustenit, 0,6 bis 1,5 % Kohlenstoff in fester Lösung, wobei der Rest Ferrit, Martensit, Bainit, Perlit und/oder Zementit ist,
- 1 bis 30 % Ferrit und 1 bis 30 % Bainit, 5 bis 25 % Austenit, wobei der Rest Martensit ist,
- 5 bis 20 % Restaustenit, wobei der Rest Martensit ist,
- Ferrit und Restaustenit,
- Restaustenit und intermetallische Phasen,
- von 80 bis 100 % Martensit und von 0 bis 20 % Restaustenit,
- 100 % Martensit,
- von 5 bis 100 % Perlit und von 0 bis 95 % Ferrit und
- mindestens 75 % gleichachsiger Ferrit, 5 bis 20 % Martensit und Bainit in einer Menge von höchstens 10 %.

5. Verfahren nach einem der Ansprüche 1 bis 4, wobei das Stahlblech Dualphasenstahl, transformationsinduzierte Plastizität, abgeschreckter und geteilter Stahl, zwillingsinduzierte Plastizität, karbidfreies Bainit, pressgehärteter Stahl, TRIPLEX, DUPLEX und hochduktiler Dualphasenstahl sein kann.

6. Verfahren nach einem der Ansprüche 1 bis 5, wobei die Unterschiede zwischen den Phasenanteilen der in $m_{Ziel}$ und $m_x$ vorhandenen Phasen $\pm 3$ % sind.

7. Verfahren nach einem der Ansprüche 1 bis 6, wobei in Schritt B.1) die zwischen $m_i$ und $m_{Ziel}$ freigesetzte oder verbrauchte thermische Enthalpie H berechnet wird, sodass:

$$H_x = (X_{Ferrit} * H_{Ferrit}) + (X_{Martensit} * H_{Martensit}) + (X_{Bainit} * H_{Bainit}) + (X_{Pearlit} * H_{Pearlit}) +$$

$$(H_{Zementit} + X_{Zementit}) + (H_{Austenit} + X_{Austenit})$$

wobei X ist ein Phasenanteil ist.

8. Verfahren nach Anspruch 7, wobei in Schritt B.1) der gesamte thermische Zyklus $TP_x$ berechnet wird, sodass:

$$T(t + \Delta t) = T(t) + \frac{(\varphi_{Convection} + \varphi_{radiance})}{\rho \cdot Ep \cdot C_{pe}} \Delta t \pm \frac{Hx}{C_{pe}}$$

wobei Cpe: die spezifische Wärme der Phase (J-kg$^{-1}$K$^{-1}$), p: die Dichte des Stahls (g.m$^{-3}$), Ep: die Stärke des Stahls (m), $\varphi$: der Wärmestrom (konvektiv + strahlend in W), $H_x$ (J.kg$^{-1}$), T: Temperatur (°C) und t: Zeit (s) ist.

9. Verfahren nach einem der Ansprüche 7 oder 8, wobei in Schritt B.1) mindestens ein Zwischenstahlgefüge $m_{xint}$, das einem thermischen Zwischenpfad $TP_{xint}$ entspricht, und die thermische Enthalpie $H_{xint}$ berechnet werden.

10. Verfahren nach Anspruch 9, wobei in Schritt B.1) $TP_x$ die Summe aller $TP_{xint}$ ist und $H_x$ die Summe aller $H_{xint}$ ist.

11. Verfahren nach einem der Ansprüche 1 bis 10, wobei vor Schritt B.1) mindestens eine angestrebte mechanische Eigenschaft $P_{Ziel}$ ausgewählt wird aus Streckgrenze YS, Zugfestigkeit UTS, Dehnungslochdehnung und Verformbarkeit.

12. Verfahren nach Anspruch 11, wobei $m_{Ziel}$ basierend auf $P_{Ziel}$ berechnet wird.

13. Verfahren nach einem der vorherigen Ansprüche, wobei in Schritt B.1) die Prozessparameter, denen das Stahlblech vor Eintritt in die Wärmebehandlungslinie unterworfen war, zur Berechnung von $TP_x$ berücksichtigt werden.

14. Verfahren nach Anspruch 13, wobei die Prozessparameter mindestens ein Element umfassen, das ausgewählt ist aus: einer Kaltwalzreduktionsrate, einer Wickeltemperatur, einem Kühlweg des Auslauftisches, einer Kühltemperatur und einer Spulenkühlrate.

15. Verfahren nach einem der Ansprüche 1 bis 14, wobei in Schritt B.1) die Prozessparameter der Behandlungslinie, die das Stahlblech in der Wärmebehandlungslinie durchlaufen wird, zur Berechnung von $TP_x$ berücksichtigt werden.

16. Verfahren nach Anspruch 15, wobei die Prozessparameter mindestens ein Element umfassen, das ausgewählt ist aus: einer spezifischen zu erreichenden Temperatur des thermischen Stahlblechs, der Liniengeschwindigkeit, der Kühlleistung der Kühlabschnitte, der Heizleistung der Heizabschnitte, einer Überalterungstemperatur, einer Kühltemperatur, einer Heiztemperatur und einer Durchwärmtemperatur.

17. Verfahren nach einem der vorhergehenden Ansprüche, wobei der thermische Weg, $TP_x$, $TP_{xint}$, TT oder $TP_{Ziel}$ mindestens eine Behandlung umfassen, die ausgewählt ist aus: einer Erwärmung, einer isothermen Behandlung oder einer Kühlung.

18. Verfahren nach einem der Ansprüche 1 bis 17, wobei jedes Mal, wenn ein neues Stahlblech in die Wärmebehandlungslinie eintritt, automatisch ein neuer Berechnungsschritt B.1) ausgeführt wird.

19. Verfahren nach Anspruch 18, wobei eine Anpassung des thermischen Pfads ausgeführt wird, wenn das Stahlblech in die Wärmebehandlungslinie auf den ersten Metern des Blechs eintritt.

20. Verfahren nach einem der Ansprüche 1 bis 19, wobei während der Wärmebehandlung eine automatische Berechnung ausgeführt wird, um zu prüfen, ob eine Abweichung aufgetreten ist.

## Revendications

1. Procédé d'ajustement dynamique pour la fabrication d'une tôle d'acier traitée thermiquement présentant une composition chimique d'acier et une microstructure $m_{cible}$ comprenant de 0 à 100 % d'au moins une phase choisie parmi la ferrite, la martensite, la bainite, la perlite, la cémentite et l'austénite, dans une ligne de traitement thermique, dans lequel un traitement thermique TT prédéfini est réalisé sur la tôle d'acier, un tel procédé comprenant :

    A. une étape de contrôle dans laquelle au moins un capteur détecte tout écart survenant pendant traitement thermique,
    B. une étape de calcul effectuée lorsqu'un écart est détecté pendant le traitement thermique, de telle sorte qu'un nouveau chemin thermique $TP_{cible}$ est déterminé pour atteindre $m_{cible}$ en tenant compte de l'écart, une telle étape de calcul comprenant :

1) une sous-étape de calcul dans laquelle au moins deux parcours thermiques, $TP_x$ correspondant à une microstructure $m_x$ obtenue à la fin de $TP_x$, sont calculés sur la base du TT et de la microstructure $m_i$ de la tôle d'acier pour atteindre $m_{cible}$,
2) une sous-étape de sélection dans laquelle un nouveau chemin thermique $TP_{cible}$ est sélectionné, visant à atteindre $m_{cible}$, $TP_{cible}$ étant choisi parmi $TP_x$ de telle sorte que $m_x$ se rapproche le plus possible de $m_{cible}$,

C. une nouvelle étape de traitement thermique dans laquelle $TP_{cible}$ est appliqué à la tôle d'acier sur la ligne de traitement.

**2.** Procédé selon la revendication 1, dans lequel, à l'étape A), l'écart est dû à une variation d'un paramètre de procédé choisi parmi : une température de four, une température de tôle d'acier, une quantité de gaz, une composition de gaz, une température de gaz, une vitesse de ligne, une défaillance dans la ligne de traitement thermique, une variation du bain d'immersion à chaud, une émissivité de la tôle d'acier et une variation de l'épaisseur de l'acier.

**3.** Procédé selon l'une quelconque des revendications 1 à 2, dans lequel les phases sont définies par au moins un élément choisi entre : la taille, la forme et la composition chimique.

**4.** Procédé selon l'une quelconque des revendications 1 à 3, dans lequel la microstructure $m_{cible}$ comprend :

- 100 % d'austénite,
- de 5 à 95 % de martensite, de 4 à 65 % de bainite, le reste étant de la ferrite,
- de 8 à 30 % d'austénite résiduelle, de 0,6 à 1,5 % de carbone en solution solide, le reste étant de la ferrite, de la martensite, de la bainite, de la perlite et/ou de la cémentite,
- de 1 % à 30 % de ferrite et de 1 % à 30 % de bainite, de 5 à 25 % d'austénite, le reste étant de la martensite,
- de 5 à 20 % d'austénite résiduelle, le reste étant de la martensite,
- de la ferrite et de l'austénite résiduelle,
- de l'austénite résiduelle et des phases intermétalliques,
- de 80 à 100 % de martensite et de 0 à 20 % d'austénite résiduelle
- 100 % de martensite,
- de 5 à 100 % de perlite et de 0 à 95 % de ferrite, et
- au moins 75 % de ferrite équiaxe, de 5 à 20 % de martensite et de bainite en quantité inférieure ou égale à 10 %.

**5.** Procédé selon l'une quelconque des revendications 1 à 4, dans lequel la tôle d'acier peut être biphasée, à plasticité induite par transformation, en acier trempé et partitionné, à plasticité induite par jumelage, en bainite sans carbure, en acier embouti à chaud, triplex, duplex et biphasée à haute ductilité.

**6.** Procédé selon l'une quelconque des revendications 1 à 5, les différences entre des proportions de phase présentes dans $m_{cible}$ et $m_x$ étant de $\pm$ 3%.

**7.** Procédé selon l'une quelconque des revendications 1 à 6, dans lequel, à l'étape B.1), l'enthalpie thermique H libérée ou consommée entre $m_i$ et $m_{cible}$ est calculée comme suit :

$$H_X = (X_{ferrite} * H_{ferrite}) + (X_{martensite} * H_{martensite}) + (X_{bainite} * H_{bainite}) + (X_{perlite} * H_{perlite})$$

$$+ (H_{cémentite} + X_{cémentite}) + (H_{austénite} + X_{austénite})$$

X étant une fraction de phase.

**8.** Procédé selon la revendication 7, dans lequel, à l'étape B.1), le cycle thermique total $TP_x$ est calculé comme suit :

$$T(t + \Delta t) = T(t) + \frac{(\varphi_{Convection} + \varphi_{radiance})}{\rho \cdot Ep \cdot C_{pe}} \Delta t \pm \frac{Hx}{C_{pe}}$$

avec Cpe : la chaleur spécifique de la phase (J.kg$^{-1}$.K$^{-1}$), p : la densité de l'acier (g.m$^{-3}$), Ep : l'épaisseur de l'acier (m), $\varphi$ : le flux thermique (convectif et radiatif, en W), H$_x$ (J.kg$^{-1}$), T : la température (°C) et t : le temps (s).

9. Procédé selon l'une quelconque des revendications 7 ou 8, dans lequel, à l'étape B.1), au moins une microstructure d'acier intermédiaire m$_{xint}$ correspondant à un chemin de refroidissement intermédiaire TP$_{xint}$ et l'enthalpie thermique H$_{xint}$ sont calculées.

10. Procédé selon la revendication 9, dans lequel, à l'étape B.1), TP$_x$ est la somme de tous les TP$_{xint}$ et H$_x$ est la somme de tous les H$_{xint}$.

11. Procédé selon l'une quelconque des revendications 1 à 10, dans lequel, avant l'étape B.1), au moins une propriété mécanique ciblée P$_{cible}$ est sélectionnée entre la limite d'élasticité (yield strength) YS, la résistance ultime à la traction (ultimate tensile strength) UTS, l'allongement, l'expansion de trou et la formabilité.

12. Procédé selon la revendication 11, dans lequel m$_{cible}$ est calculé sur la base de P$_{cible}$.

13. Procédé selon l'une quelconque des revendications précédentes, dans lequel, à l'étape B.1), les paramètres de traitement auxquels est soumise la tôle d'acier avant de rejoindre la ligne de traitement thermique sont pris en compte pour calculer TP$_x$.

14. Procédé selon la revendication 13, dans lequel les paramètres de traitement comprennent au moins un élément choisi parmi : un taux de réduction par laminage à froid, une température d'enroulement, un chemin de refroidissement de table de sortie, une température de refroidissement et une vitesse de refroidissement de bobine.

15. Procédé selon l'une quelconque des revendications 1 à 14, dans lequel, à l'étape B.1), les paramètres de traitement de la ligne de traitement auxquels sera soumise la tôle d'acier dans la ligne de traitement thermique sont pris en compte pour calculer TP$_x$.

16. Procédé selon la revendication 15, dans lequel les paramètres de traitement comprennent au moins un élément choisi parmi : une température spécifique de la tôle d'acier à atteindre, la vitesse de la ligne, la puissance de refroidissement des sections de refroidissement, la puissance de chauffage des sections de chauffage, une température de sur-vieillissement, une température de refroidissement, une température de chauffage et une température de trempage.

17. Procédé selon l'une quelconque des revendications précédentes, dans lequel le chemin thermique, TP$_x$, TP$_{xint}$, TT ou TP$_{cible}$ comprend au moins un traitement choisi parmi : un traitement de chauffage, un traitement isotherme ou un traitement de refroidissement.

18. Procédé selon l'une quelconque des revendications 1 à 17, dans lequel, chaque fois qu'une nouvelle tôle d'acier est introduite dans la ligne de traitement thermique, une nouvelle étape de calcul B.1) est automatiquement effectuée.

19. Procédé selon la revendication 18, dans lequel une adaptation du chemin thermique est réalisée lors de l'entrée de la tôle d'acier dans la ligne de traitement thermique sur les premiers mètres de la tôle.

20. Procédé selon l'une quelconque des revendications 1 à 19, dans lequel un calcul automatique est effectué pendant le traitement thermique pour vérifier si un écart apparaît.

## Figure 1

**Figure 2**

# Figure 3

$$TT, m_{target}, CC, m_i$$

Control step A)

Deviation detected

Calculation step B.1)

$$TP_{Xint1}, m_{Xint1}, H_{Xint1}$$

Calculation step

$$TP_{Xint2}, m_{Xint2} \, H_{Xint2}$$

Calculation step

$$TP_{X,} m_x$$

Selection step B.2)

$$TP_{target} \text{ to reach } m_{target}$$

**Figure 4**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4440583 A
- US 2009265146 A